# EUROPEAN PATENT SPECIFICATION

(11) **EP 1 593 746 B1**
(45) Date of publication and mention of the grant of the patent: **09.01.2013**
(21) Application number: 04710967.3
(22) Date of filing: 13.02.2004
(51) Int. Cl.: C12Q 1/68

(54) **SIGNAL AMPLIFICATION METHOD FOR DETECTING EXPRESSED GENE**
SIGNALVERSTÄRKUNGSVERFAHREN ZUM NACHWEIS EINES EXPRIMIERTEN GENS
PROCEDE D'AMPLIFICATION DE SIGNAUX DESTINE A LA DETECTION DU GENE EXPRIME

(30) Priority: 14.02.2003 JP 2003037212
(43) Date of publication of application: 09.11.2005
(73) Proprietor: Eisai R&D Management Co., Ltd., Bunkyo, Tokyo (JP)
(72) Inventor: USUI, Mitsugu, No. 507, Kikuna Residencia Plaza, Yokohama-shi, Kanagawa (JP); FUJIKAWA, Toshihiko, Kawasaki-shi, Kanagawa 212-0052 (JP)
(74) Representative: Grünecker, Kinkeldey, Stockmair & Schwanhäusser
(86) International application number: PCT/JP2004/001588
(87) International publication number: WO 2004/072302

(56) References cited:
- EP-A- 1 229 131
- WO-A1-01/66555
- WO-A1-02/18642
- WO-A1-95/21271
- JP-A- 2002 355 081
- JP-A- 2002 355 081
- JP-A- 2003 061 679
- US-A1- 2003 008 294
- US-B1- 6 261 846
- VANDEVYVER C ET AL: "Quantitative analysis of lymphokine mRNA expression by an automated, non-radioactive method." CELLULAR AND MOLECULAR BIOLOGY (NOISY-LE-GRAND, FRANCE) JUL 1995, vol. 41, no. 5, July 1995 (1995-07), pages 683-694, XP009091310 ISSN: 0145-5680
- TANIGUCHI A ET AL: "Competitive RT-PCR ELISA: A rapid, sensitive and non-radioactive method to quantitate cytokine mRNA" JOURNAL OF IMMUNOLOGICAL METHODS, ELSEVIER SCIENCE PUBLISHERS B.V.,AMSTERDAM, NL, vol. 169, 1994, pages 101-109, XP002115774 ISSN: 0022-1759
- FOLEY KEVIN P ET AL: "Quantitation of RNA using the polymerase chain reaction" TRENDS IN GENETICS, vol. 9, no. 11, 1993, pages 380-385, XP002455930 ISSN: 0168-9525
- HURTEAU G J ET AL: "mRNA-specific reverse transcription-polymerase chain reaction from human tissue extracts", ANALYTICAL BIOCHEMISTRY, ACADEMIC PRESS INC, NEW YORK, vol. 307, no. 2, 15 August 2002 (2002-08-15), pages 304-315, XP002324197, ISSN: 0003-2697, DOI: 10.1016/S0003-2697(02)00058-1
- NILSEN T W ET AL: "DENDRITIC NUCLEIC ACID STRUCTURES", JOURNAL OF THEORETICAL BIOLOGY, ACADEMIC PRESS, LONDON, GB, vol. 187, 1 January 1997 (1997-01-01), pages 273-284, XP002940183, ISSN: 0022-5193, DOI: 10.1006/JTBI.1997.0446

### SEQUENCE LISTING

<110> Eisal Co., Ltd.
<120> Signal Amplification Method for Detecting Expressed Gene
<130> 76423-PCT-EP
<140> PCT/JP2004/001588
   <141> 2004-02-13
<150> JP 2003-037212
   <151> 2003-02-14
<160> 6
<210> 1
   <211> 24
   <212> DNA
   <213> Artificial Sequence
<220>
   <223> Description of Artificial Sequence: CP-1
<400> 1
   cacgaaacta ccttcaactc catc 24
<210> 2
   <211> 21
   <212> DNA
   <213> Artificial Sequence
<220>
   <223> Description of Artificial Sequence: CP-2
<400> 2
   tgccgacagg atgcagaagg a 21
<210> 3
   <211> 78
   <212> DNA
   <213> Artificial Sequence
<220>
   <223> Description of Artificial Sequence: first probe
<400> 3
   gcatatagat atctccggcg cggatacttt gtgataccgg gagttcgccc ttataacgtc 60
tttttttttt tttttttt 78
<210> 4
   <211> 18
   <212> DNA
   <213> Artificial Sequence
<220>
   <223> Description of Artificial Sequence: poly dT primer
<400> 4
   tttttttttt tttttttt 18
<210> 5
   <211> 60
   <212> DNA
   <213> Artificial Sequence
<220>
   <221> misc_feature
   <222> (1)
   <223> Cy3 attached at the 5'end
<400> 5
   cgccggagat atctatatgc ccggtatcac aaagtatccg gacgttataa gggcgaactc 60
<210> 6
   <211> 60
   <212> DNA
   <213> Artificial Sequence
<220>
   <221> misc_feature
   <222> (1)
   <223> Cy3 attached at the 5'end
<220>
   <223> Description of Artificial Sequence: HCP-2
<400> 6
   gcatatagat atctccggcg cggatacttt gtgataccgg gagttcgccc ttataacgtc 60

## Description

The present invention relates to a method for detecting an expressed gene and more specifically relates to a method for detecting an expressed gene which, by the use of a reverse transcription reaction and a self-assembly reaction of a pair of honeycomb probes, is capable of improving detection sensitivity and detecting a target gene depending on the length and expression amount of a target RNA.

Usually, in a detecting method for expressed genes with DNA chips, using a primer having only poly(dT) or a random primer, by a reverse transcription reaction, a labeled cDNA in which a nucleic acid labeled with a fluorescent material such as Cy3 and Cy5 is incorporated turns to a probe. When very small amounts of samples are used, anti-sense RNA is generally synthesized using a linear amplification method (for example, see "Practical Manual of DNA Microarray" supervised by Yoshihide HAYASHIZAKI, issued by YODOSHA CO., LTD., December 1, 2000, pp. 80-90). The linear amplification method includes: synthesizing a first cDNA strand; then synthesizing a second cDNA strand using three enzymes of RNase H, DNA polymerase I and DNA ligase; and finally performing a transcription reaction in vitro with RNA polymerase to amplify anti-sense RNA. However, the linear amplification method has the disadvantage that it needs various types of expensive enzymes and involves a very complicated operation. If the amount of samples is extremely small, the linear amplification method must be performed twice or more. There is also a problem that the anti-sense RNA produced by the linear amplification method tends to be shorter than the original RNA so that the length of the original RNA cannot precisely be reflected.

The present inventors have reported a new isothermal nucleic acid amplification method without using any enzyme (for example, see USP 6,261,846, JP 3267576, and EP 1,002,877A). This method uses a pair of oligonucleotides each comprising three regions (Honeycomb Probe, hereinafter referred to as an HCP) in which the three respective regions of a first HCP and a second HCP are designed to be composed of base sequences complementary to each other so that only one region of the first HCP may be hybridized with one region of the second HCP when the two oligonucleotides are reacted. This design makes it possible for a plurality of pairs of the HCPs to hybridize to each other and form an assembly substance by a self-assembly reaction of the HCPs when they are reacted to each other (Probe Alternation Link Self-Assembly Reaction; this method for the formation of an assembly substance by the self-assembly reaction of the HCPs is referred to as a PALSAR method hereinafter).

US 2003/0008294 describes a method for detecting a target gene comprising the use of honeycomb probes. However, US 2003/0008294 does not describe a method including an RT step and further, does not describe the use of a single type of RT primer allowing the simultaneous detection of different target genes.

WO 01/66555 describes a method for detecting an expressed gene using prehybridized cDNA-dendrimer complexes.

### Disclosure of the Invention:

It is an object of the present invention to provide a method for detecting an expressed gene, which can realize detection with an inexpensive and simple operation in a short time without expensive enzymes and can realize detection depending on the length and expressed amount of the original RNA without the use of the linear amplification method or the PCR method.

In order to solve the above problem, in a first aspect the following method is provided:

A method for detecting an expressed gene, wherein the detection sensitivity of the expressed gene on a DNA chip is improved by the use of a reverse transcription reaction and a self-assembly reaction forming a self-assembly substance by means of self-assembling of a pair of honeycomb probes, the method comprising the steps of:
performing a reverse transcription reaction of mRNA using a first probe containing poly(dT) at the 3' end and a region hybridizable with one of the honeycomb probes as a primer to form a second probe having a cDNA region;
separating the mRNA from the second probe;
hybridizing the second probe with a capture probe having a region complementary to a cDNA region of a target mRNA;
forming a self-assembly substance by a self-assembly reaction using the second probe and the honeycomb probes, wherein the honeycomb probes self-assemble to form the self-assembly substance; and
detecting the presence of the self-assembly substance.

While any special limitation is not put on the stage where the step for forming the self-assembly substance by the self-assembly reaction, the forming step is preferably carried out after the step for hybridizing the second probe with the capture probe.

In one aspect, the self-assembly reaction may be performed using a plurality of pairs of honeycomb probes, in which the number of base sequence regions complementary each other is n (n≥3), in such a manner that by hybridizing the honeycomb probes each other in alternation, the honeycomb probes self-assemble to form a double-stranded self-assembly substance.

In another aspect, the self-assembly reaction may comprise the steps of:
providing a first group and a second group of probes,
the first group including a plurality of pairs of dimer-forming honeycomb probes containing a pair of an oligonucleotide No.1 and an oligonucleotide No.2, each oligonucleotide having three regions of a 3' side region, a mid-region and a 5' side region, in which the mid-regions thereof have base sequence complementary to each other to form a dimer probe, and the 3' side regions and the 5' side regions thereof have base sequences not complementary to each other, and
the second group including a plurality of pairs of cross-linking probes containing a pair of an oligonucleotide No.3 and an oligonucleotide No.4, each oligonucleotide having two regions of a 3' side region and a 5' side region, in which the 3' side regions and the 5' side regions thereof have base sequences not complementary to each other, and the pairs of the cross-linking probes having base sequences capable of cross-linking the dimer probes formed from the dimer-forming probes; and
hybridizing the probes,
wherein the probes self-assemble to form the self-assembly substance.

The base sequences of the probes may be complementary to each other in the following respective pairs:,
the 3' side region of the oligonucleotide No.1 in the first group and
the 3' side region of the oligonucleotide No.3 in the second group;
the 5' side region of the oligonucleotide No.2 in the first group and
the 5' side region of the oligonucleotide No.4 in the second group;
the 3' side region of the oligonucleotide No.4 in the second group and
the 3' side region of the oligonucleotide No.2 in the first group; and
the 5' side region of the oligonucleotide No.3 in the second group and
the 5' side region of the oligonucleotide No.1 in the first group.

The base sequences of the probes may be complementary to each other in the following respective pairs:,
the 3' side region of the oligonucleotide No.1 in the first group and
the 3' side region of the oligonucleotide No.3 in the second group;
the 5' side region of the oligonucleotide No.2 in the first group and
the 5' side region of the oligonucleotide No.3 in the second group;
the 3' side region of the oligonucleotide No.2 in the first group and
the 3' side region of the oligonucleotide No.4 in the second group; and
the 5' side region of the oligonucleotide No.1 in the first group and
the 5' side region of the oligonucleotide No.4 in the second group.

In a further aspect, the method for detecting an expressed gene according to the present invention is characterized in that the detection sensitivity of the expressed gene on a DNA chip is improved by the use of a reverse transcription reaction and a self-assembly reaction, wherein the self-assembly reaction is performed using a plurality of pairs of a first honeycomb probe (HCP) and a second HCP, in which the number of base sequence regions complementary each other is n (n≥3), in such a manner that by hybridizing the honeycomb probes to each other in alternation, the honeycomb probes self-assemble to form a double-stranded self-assembly substance, the method comprising the steps of:
binding a first probe containing poly(dT) at the 3' end and at least a part of the base sequence regions of the first HCP to mRNA;
performing a reverse transcription reaction by a reverse transcriptase to form a second probe containing a cDNA region and at least a part of the base sequence regions of the first HCP;
removing the mRNA; thereafter
hybridizing the second probe with a capture probe having a region complementary to a cDNA region of a target mRNA;
adding both the first HCP and the second HCP or adding the second HCP to form a self-assembly substance by the self-assembly reaction of the probes so that signal amplification can be achieved; and
detecting the presence of the self-assembly substance.

In the method of the present invention, there may be employed mRNA containing poly(A) at the end thereof as a target expressed gene.

It is preferable that the DNA chip has a support to which the capture probe for capturing the target gene is bound and the support is a microplate type, a slide glass type, a particle type, or an electroconductive substrate type. The support of the microplate type or the particle type may be made of plastics such as polystyrene. Materials such as glass or plastics may be employed for the support of the slide glass type. A gold electrode, an ITO (indium oxide) electrode or the like may be used for the support of the electroconductive substrate type.

A labeled probe may be hybridized with the self-assembly substance so that the presence of the self-assembly substance can be detected.

The labeled probe is preferably a probe labeled with an enzyme of color generation type, an enzyme of luminescence generation type or a radioisotope.

The presence of the self-assembly substance may be detected by:
adding a fluorescent substance capable of binding to a nucleic acid to the self-assembly substance; and
measuring a photochemical change of the fluorescent substance.

The presence of the self-assembly substance may be detected by:
labeling in advance at least one of the honeycomb probes forming the self-assembly substance with a fluorescent substance; and
measuring a photochemical change of the fluorescent substance.

The presence of the self-assembly substance may be detected by:
labeling in advance at least one of the honeycomb probes forming the self-assembly substance with a radioisotope; and
detecting the radioisotope.

The presence of the self-assembly substance may be detected by:
labeling in advance at least one of the honeycomb probes forming the self assembly substance with an enzyme of color generation type or an enzyme of luminescence generation type; and
measuring a photochemical change due to the enzyme.

The honeycomb probes may be comprised of at least one base selected from the group consisting of DNA, RNA, PNA, and LNA.

### Brief Description of the Drawings:

Fig. 1 is a flow chart showing an example of order of steps of a method according to the present invention;
Fig. 2 is a schematic diagram showing in principle the step 200 in a first example of order of steps of the method according to the present invention;
Fig. 3 is a schematic diagram showing in principle the step 202 in the first example of order of steps of the method of the present invention;
Fig. 4 is a schematic diagram showing in principle the step 204 in the first example of order of steps of the method of the present invention;
Fig. 5 is a schematic diagram showing in principle the step 206 in the first example of order of steps of the method of the present invention;
Fig. 6 is a schematic diagram showing in principle the step 210 in the first example of order of steps of the method of the present invention;
Fig. 7 is a schematic diagram showing in principle the step 212 in the first example of order of steps of the method of the present invention;
Fig. 8 is a schematic diagram showing in principle the step 214 in the first example of order of steps of the method of the present invention;
Fig. 9 is a schematic diagram showing in principle the step 300 in a second example of order of steps of the method of the present invention;
Fig. 10 is a schematic diagram showing in principle the step 302 in the second example of order of steps of the method of the present invention;
Fig. 11 is a schematic diagram showing in principle the step 304 in the second example of order of steps of the method of the present invention;
Fig. 12 is a schematic diagram showing in principle the step 306 in the second example of order of steps of the method of the present invention; and
Fig. 13 is a graph showing the results of Example 1 and Comparative

### Example 1.

### Best Mode for Carrying Out the Invention:

The examples of the present invention are described below with reference to the attached drawings. It should be understood that the examples described herein are merely exemplary.

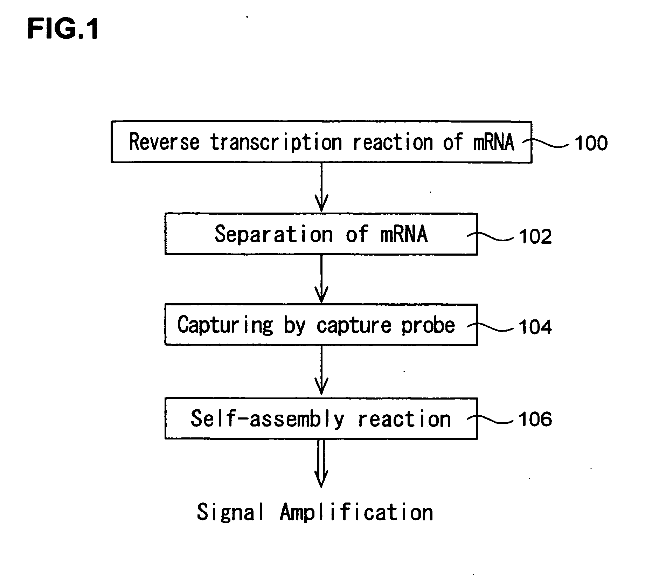

Fig. 1 is a flow chart showing an example of order of steps of the method for detecting an expressed gene according to the present invention.

As shown in Fig. 1, there is provided a first probe which contains poly(dT) at the 3' end and a region hybridizable with at least one honeycomb probe for use in a self-assembly reaction. The first probe is used as a primer and bound to mRNA containing poly(A). A reverse transcription reaction of the mRNA is performed by a reverse transcriptase (step 100) so that there is formed a second probe which is comprised of the first probe and a cDNA region of the mRNA. In a preferred mode, a base sequence in the 5' side of the first probe includes at least a part of the base sequence of the honeycomb probe for use in the self-assembly reaction.

The mRNA is then separated from the second probe (step 102). Any limitations are not specifically put on the method for separating the mRNA, but methods using, for example, thermal denaturation, alkali denaturation, RNA digestion with RNase H, or the like may be employed.

After the separation, the second probe is hybridized with a capture probe having a region complementary to a cDNA region of a target mRNA to allow the capture probe to capture the second probe (step 104). Preferably, the capture probe has previously been bound to a support.

The honeycomb probes are added so that a self-assembly substance hybridized with the second probe can be formed by a self-assembly reaction (step 106) with the result that the signal can be amplified.

If the target mRNA does not exist in the sample, the second probe cannot bind to the capture probe so that signal amplification cannot be carried out. Thus, the existence of the target mRNA can be determined by the method of the present invention. Since the method of the present invention does not use the linear amplification method, detection can be performed depending on the length of the original RNA, and since the method of the present invention does not use the PCR method, signal amplification can be performed depending on the amount of expression.

As the self-assembly reaction described above, there may be employed a self-assembly reaction using a pair of HCPs where each of the HCPs comprises three regions complementary to each other and the HCPs can self-assemble by themselves to form a self-assembly substance (for example, see JP 3267576 and JP 3310662). Alternatively, a self-assembly reaction may be performed using a pair of dimer-forming probes capable of forming a dimer by themselves and a pair of crosslinking probes capable of crosslinking the dimer formed from the dimer-forming probes (for example, see JP-A 2002-355081).

Although step 104 is followed by step 106 in the example shown in Fig. 1, step 106 may be performed before or simultaneously with step 104.

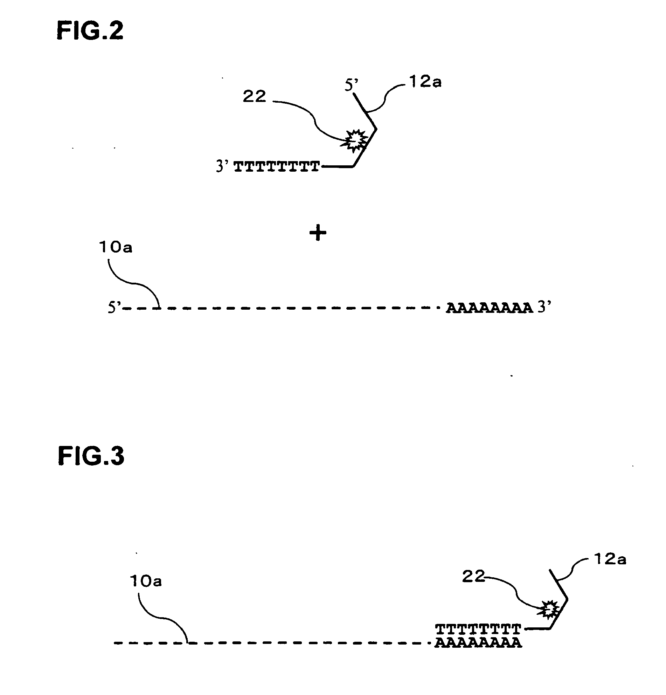

Figs. 2 to 8 are schematic diagrams showing in principle a first example of order of steps of the method according to the present invention. The first example illustrates the signal amplification method utilizing the PALSAR method using a pair of HCPs previously labeled with a fluorescent material 22 as the self-assembly reaction, wherein an HCP containing poly(dT) at the 3' end is used as a first probe 12a.

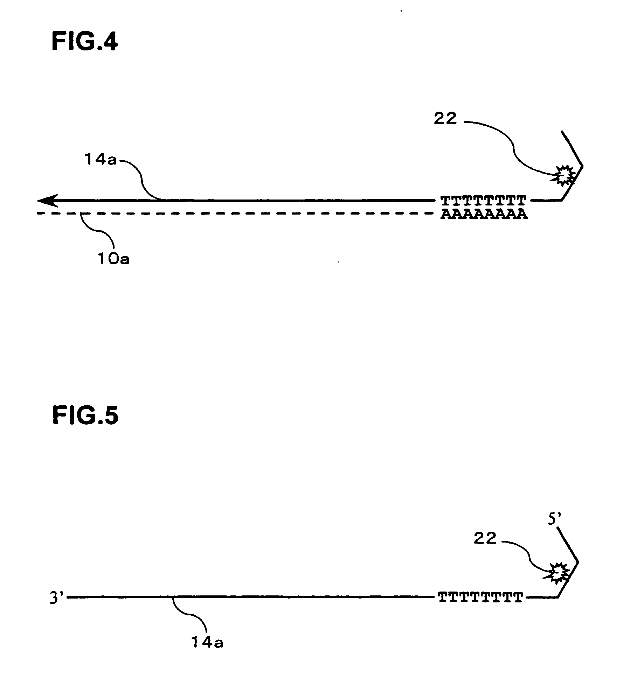

As shown in Fig. 2, in order to detect mRNA 10a of a target gene, a honeycomb probe (HCP-1) containing poly(dT) at the 3' end and three regions of the HCP in the 5' side thereof is provided as the first probe 12a (step 200). As shown in Fig. 3, HCP-1 (12a) containing poly(dT) at the 3' end is bound to the poly(A) tail part of the mRNA 10a (step 202). As shown in Fig. 4, a reverse transcription reaction is then performed using a reverse transcriptase to form a second probe 14a having a sequence complementary to the mRNA (step 204). Thereafter, as shown in Fig. 5, the mRNA 10a is dissociated to form a single-stranded oligonucleotide comprising a cDNA region and the HCP region (step 206).

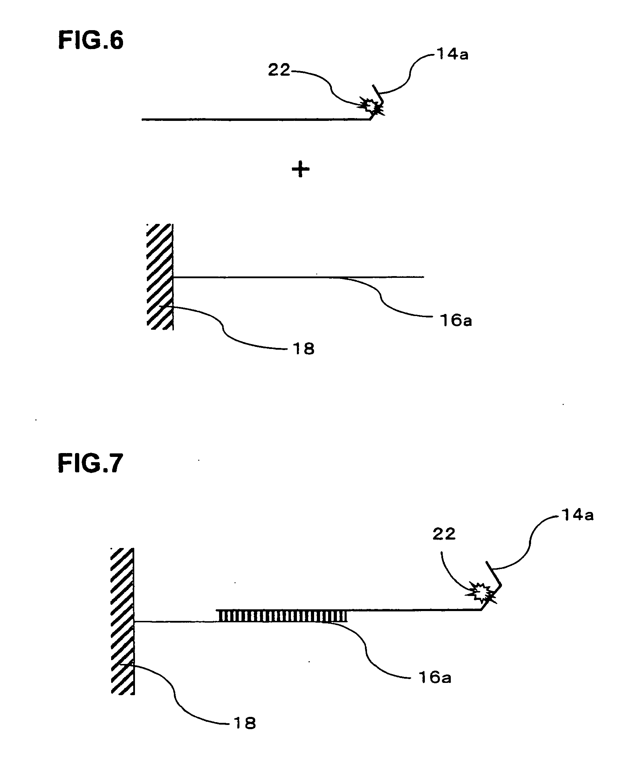

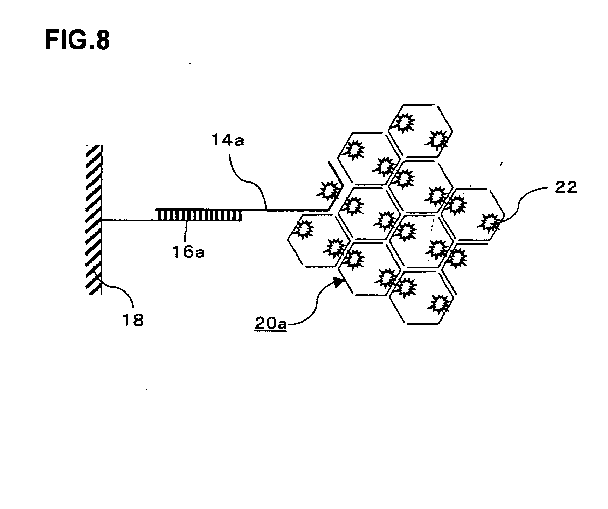

As shown in Fig. 6, a capture probe 16a having a region complementary to the cDNA of the target gene is previously bound to a support 18 (step 210). As shown in Fig. 7, the second probe 14a serving as an HCP having the formed cDNA region is hybridized with the capture probe 16a (step 212). As shown in Fig. 8, another HCP (HCP-2) of the pair of HCPs is added to form a self-assembly substance 20a by a self-assembly reaction (step 214) so that signal amplification can be achieved. Incidentally, if in the step 206, a washing operation is carried out when the target mRNA is removed, the pair of HCPs must be added in the step 214.

In the above first example, there is used a pair of HCPs, wherein a part of the 3' side region in the three regions of HCP-1 is poly(dT). Alternatively, there may be used another pair of HCPs, wherein the 3' side region of HCP-1 is entirely poly(dT) and the 3' side region in the three regions of HCP-2 is entirely poly(A).

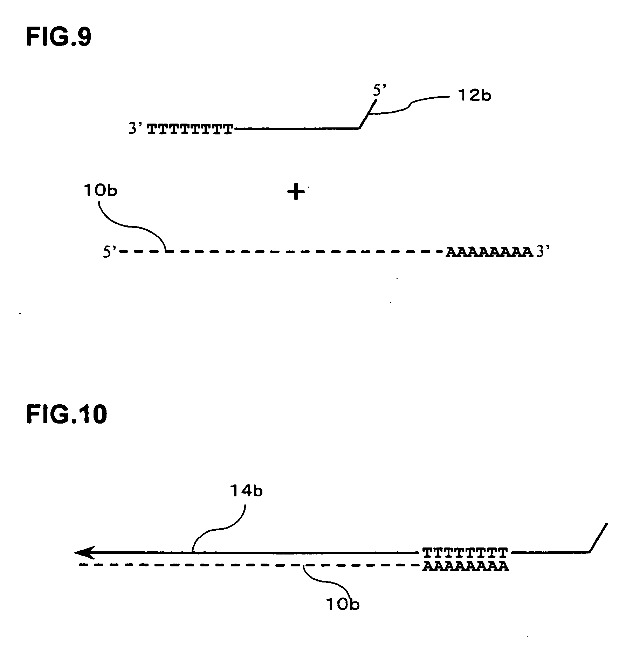

Figs. 9 to 12 are schematic diagrams showing in principle a first example of order of steps of the method according to the present invention. The second example illustrates the method utilizing the PALSAR method using a pair of HCPs unlabeled with a fluorescent material as the self-assembly reaction, wherein a probe containing poly(dT) at the 3' end and one of complementary regions of the HCP is used as a first probe 12b.

As shown in Fig. 9, in order to detect mRNA 10b of a target gene, a probe containing poly(dT) at the 3' end and one of the complementary regions of the HCP in the 5' side thereof is provided as the first probe 12b (step 300). As shown in Fig. 10, the probe 12b is bound to the poly(A) tail part of the mRNA 10b, and a reverse transcription reaction is performed using a reverse transcriptase to form a second probe 14b having a sequence complementary to the mRNA (step 302). The mRNA 10b is separated to form a single-stranded oligonucleotide comprising a cDNA region and the HCP region.

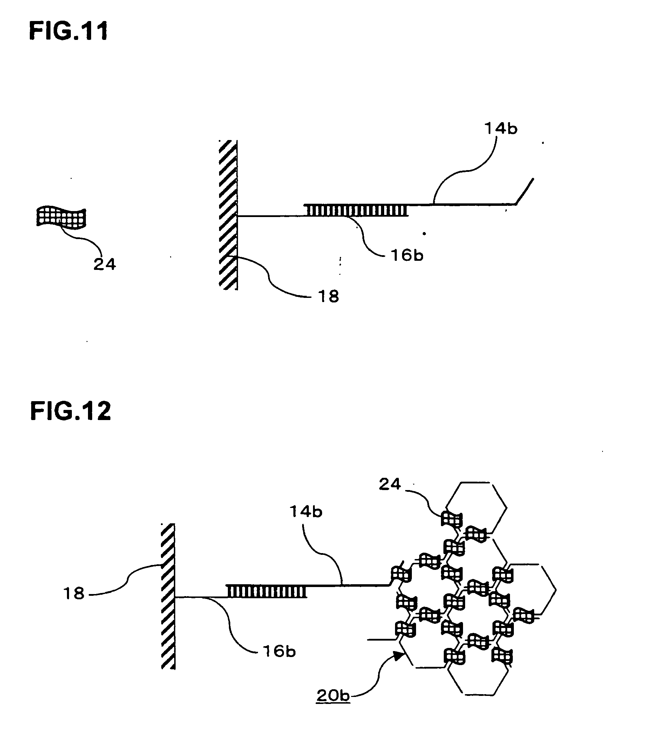

As shown in Fig. 11, the second probe 14b is hybridized with a capture probe 16b bounded to a support 18 (step 304). As shown in Fig. 12, a pair of HCPs is added to form a self-assembly substance 20b by a self-assembly reaction (step 306). An intercalator 24 or the like is inserted into the formed self-assembly substance 20b (step 308) so that signal amplification can be achieved. Steps 306 and 308 may be performed simultaneously.

For detection of the target gene, a labeling material for detection may previously be added to the pair of the honeycomb probes. Examples of such a labeling material include radioisotopes such as I¹²⁵ and P³², luminescent materials such as digoxigenin and acridinium esters, fluorescent materials such as Cy3 and Cy5, and fluorescent donor dyes and fluorescent acceptor dyes for using fluorescent resonance energy transfer (FRET) such as biotin for using a fluorescent material such as 4-methylunbelliferyl phosphate.

Alternatively, by adding a dye having the property of binding to nucleic acids, the target gene can be detected. A fluorescent material having the property of binding to nucleic acids, such as an intercalator, is preferably used to detect the target gene. Any fluorescent material having the property of binding to nucleic acids may be used without limitation. Examples of such a fluorescent material include SYBR Green I stain, SYBR Green II stain, SYBR Green Gold stain, Vistra Green stain, Gelstar stain, Radlant Red stain, PicoGreen, RiboGreen, OllGreen, Hoechst 33258 (Bis-Benzimide), Propidium Iodide, YO-PRO-1 Iodide, YO-PRO-3 Iodide (the above materials are all manufactured by Molecular Probes Inc.), ethidium bromide, Distamycin A, TOTO, Psoralen, acridinium orange (Acridine Orange), AOAO (homodimer), and the like.

While a nucleic acid constituting the pair of the honeycomb probes is usually DNA or RNA, a nucleic acid analogue may constitute them. Examples of such a nucleic acid analogue include peptide nucleic acids (PNAs, for example, see the brochure of International Patent Publication No. WO 92/20702) and locked nucleic acids (LNAs, for example, see Koshkin AA et al., Tetrahedron 1998, 54, 3607-3630, Koshkin AA et al., J. Am. Chem. Soc., 1998, 120, 13252-13253 and Wahlestedt C et al., PNAS, 2000, 97, 5633-5638). The pair of the honeycomb probes is generally composed of nucleic acids of the same kind, but may be composed of a pair of a DNA probe and an RNA probe. That is, the type of the nucleic acid of the probe may be selected from DNA, RNA or nucleic acid analogues (such as PNA and LNA). Also, it is not necessary that a single probe is composed of a single type of nucleic acid, for example, DNA only, and, if necessary, for example, a honeycomb probe composed of DNA and RNA (a chimera probe) may be used in an aspect of the present invention.

In terms of the number of bases, the length of each complementary base sequence region of the honeycomb probe may be at least 5 bases, preferably from 10 to 100 bases, more preferably from 15 to 30 bases.

These probes may be synthesized by any known methods. For example, DNA probes may be synthesized by a phosphoamidite method using DNA Synthesizer Model 394 (Applied Biosystems Inc.). Any other synthesis methods may also be used such as a phosphotriester method, an H-phosphonate method, and a thiophosphonate method.

According to the present invention, a self-assembly substance is formed with a pair of HCPs having complementary regions against a target gene captured on a DNA chip. While the number of pieces of the honeycomb probe for use is not limited, it may be in the range of 10² to 10¹⁵. The reaction buffer solution may have any composition and any concentration, and any buffer solution commonly used for nucleic acid amplification may be preferably used. The pH may be in any conventional range, preferably in the range of 7.0 to 9.0. The reaction temperature may be from 40 to 80°C, preferably from 55 to 65°C.

In the present invention, any sample potentially containing a target nucleic acid may be used as a sample for the measurement of a target expressed gene (mRNA). The target gene may be any properly prepared or isolated from samples and it is not specifically limited. Examples of such samples include organism-derived samples such as blood, blood serum, urine, feces, cerebrospinal fluid, tissue fluid, and cell cultures, and any samples potentially containing or potentially infected with any eukaryote having mRNA containing a poly(A) strand at its end, such as fungi. There may be also used any nucleic acid obtained by amplifying a target gene in samples with any known method.

### (Examples)

Although the present invention is more specifically described by means of the examples below, it will be understood that the examples presented are by way of illustration only and should not be construed as any limitation on the present invention.

The materials below were used in the examples.
(a) Target gene: total RNA extracted from cultured cells
(b) Capture probe:
   1) CP-1: 5'-CACGAAACTACCTTCAACTCCATC-3'
   2) CP-2: 5'-TGCCGACAGGATGCAGAAGGA-3'
(c) Primers:
   1) First probe (poly(dT)-HCP, 78 mer): 5'-GCATATAGATATCTCC GGCGCGGATACTTTGTGATACCGGGAGTTCGCCCTTATAACGTCTTTTT TTTTTTTTTTTT-3'
   2) Poly(dT) primer (18 mer): 5'-TTTTTTTTTTTTTTTTT-3' (d) HCPs:
      1) HCP-1 (Cy3-labeled 5' end, 60 mer): 5'-Cy3-CGCCGGAGATAT CTATATGCCCGGTATCACAAAGTATCCGGACGTTATAAGGGCGAACTC-3'
      2) HCP-2 (Cy3-labeled 5' end, 60 mer): 5'-Cy3-GCATATAGATATC TCCGGCGCGGATACTTTGTGATACCGGGAGTTCGCCCTTATAACGTC-3' (e) Polystyrene particle beads: a single kind of particle beads having the above two kinds of capture probes fixed thereon.

### (Example 1)

Using the total RNA extracted from cultured cells, an attempt was made to detect beta-actin of a housekeeping gene according to the PALSAR method.

### (1) Reverse Transcription Reaction of RNA and Purification of Reverse Transcription Product

A reverse transcription reaction was carried out at 37°C for 2 hours using the target gene, the first probe, a reverse transcriptase (SuperScript II manufactured by Invitrogen Corporation), and a reaction solution (Reaction Buffer, DTT, dNTP, RNase inhibitor). Thereafter, alkali treatment was performed at 65°C for 30 minutes, and neutralization was performed using hydrochloric acid. The reverse transcription product of cDNA was purified using QIAquick PCR Purification Kit (manufactured by QIAGEN K. K.).

### (2) Hybridization

The obtained cDNA, the particle beads, 6x SSC, 0.2% SDS, and 5x Denhardt's solution, were then mixed to prepare a composition of 50 µl in total amount. The composition was subjected to hybridization at 42°C for 2 hours. After the hybridization was completed, filtration was performed with a 0.22 µm filter so that the unreacted probe was removed. Then the particle beads was cleaned once with 2x SSC + 0.1% SDS and once with 0.2x SSC, and was filtered with the above filter.

Thereafter, a composition of 100 µl in total amount consisting of the obtained particle beads, the HCPs-1 and 2 (each 1.5 pmol/µl), 1% Blocking Reagent (manufactured by Roche Inc.), 0.1% N-lauroylsarcosine, 0.02% SDS, and 5x SSC was subjected to hybridization at 65°C for 30 minutes.

### (3) Detection

After washing, the particle beads were resuspended in sheath fluid for a flow cytometer, and the fluorescence of the Cy3 labeled to the HCPs was measured with the flow cytometer.

### (Comparative Example 1)

The target gene was detected using a conventional Cy3-dNTP uptake system with a poly(dT) primer.

### (1) Reverse Transcription Reaction and Separation Reaction of RNA

The reverse transcription reaction and the purification of the reverse transcription product were performed under the same process as in Example 1 with the exception of using the poly(dT) primer as primer, and introducing Cy3-labeled dUTP (manufactured by Amersham Inc.) in addition to dNTP.

### (2) Hybridization

The obtained cDNA, the particle beads, 6x SSC, 0.2% SDS, and 5x Denhardt's solution were then mixed to prepare a composition of 50 µl in total amount. The composition was subjected to hybridization at 42°C for 2 hours. After the hybridization was completed, filtration was performed with a 0.22 µm filter so that the unreacted probe was removed. Then the composition was cleaned once with 2x SSC + 0.1% SDS, and was filtered.

### (3) Detection

After washing, the particle beads were resuspended in a sheath fluid for a flow cytometer, and the fluorescence of the Cy3 label of the cDNA bound to the capture probe on the particle beads was measured with the flow cytometer.

### [Results]

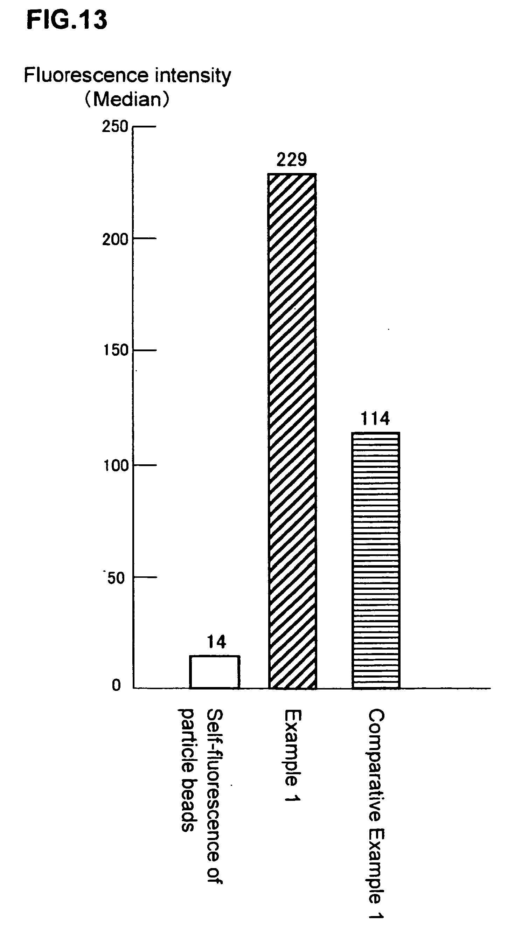

The results of Example 1 and Comparative Example 1 are shown in Fig. 13. The fluorescence intensity was measured for 203 to 504 particle beads in respect of each one and indicated by the median thereof. As shown in Fig. 13, the detection sensitivity of Example 1 is significantly higher than that of Comparative Example 1.

### Capability of Exploitation in Industry:

As described above, according to the present invention, the detection only through a reverse transcription reaction can be accomplished by an inexpensive and simple operation in a short time without expensive enzymes. A further significant advantage is that since there is no need to use the linear amplification method or the PCR method, the detection can also be performed depending on the length or expression amount of the original RNA.

## Claims

1. A method for detecting an expressed gene, wherein the detection sensitivity of the expressed gene on a DNA chip is improved by the use of a reverse transcription reaction and a self-assembly reaction forming a self-assembly substance by means of self-assembling of a pair of honeycomb probes, the method comprising the steps of:
performing a reverse transcription reaction of mRNA using a first probe containing poly(dT) at the 3' end and a region hybridizable with one of the honeycomb probes as a primer to form a second probe having a cDNA region;
separating the mRNA from the second probe;
hybridizing the second probe with a capture probe having a region complementary to a cDNA region of a target mRNA;
forming a self-assembly substance by a self-assembly reaction using the second probe and the honeycomb probes, wherein the honeycomb probes self-assemble to form the self-assembly substance; and
detecting the presence of the self-assembly substance.

2. The method according to claim 1, wherein the self-assembly reaction is performed using a plurality of pairs of honeycomb probes, in which the number of base sequence regions complementary to each other is n (n≥3), in such a manner that by hybridizing the honeycomb probes to each other in alternation, the honeycomb probes self-assemble to form a double-stranded self-assembly substance.

3. A method for detecting an expressed gene, wherein the detection sensitivity of the expressed gene on a DNA chip is improved by the use of a reverse transcription reaction and a self-assembly reaction forming a self-assembly substance by means of self-assembling of a pair of honeycomb probes, wherein the self-assembly reaction is performed using a plurality of pairs of a first honeycomb probe (HCP) and a second HCP, in which the number of base sequence regions complementary to each other is n (n≥3), in such a manner that by hybridizing the honeycomb probes to each other in alternation, the honeycomb probes self-assemble to form a double-stranded self-assembly substance, the method comprising the steps of:
binding a first probe containing poly(dT) at the 3' end and at least a part of the base sequence regions of the first HCP to mRNA;
performing a reverse transcription reaction by a reverse transcriptase to form a second probe containing a cDNA region and at least a part of the base sequence regions of the first HCP;
removing the mRNA; thereafter
hybridizing the second probe with a capture probe having a region complementary to a cDNA region of a target mRNA;
adding both the first HCP and the second HCP or adding the second HCP to form a self-assembly substance by the self-assembly reaction of the honeycomb probes so that signal amplification can be achieved; and
detecting the presence of the self-assembly substance.

4. The method according to claim 1, wherein the self-assembly reaction comprises the steps of:
providing a first group and a second group of probes,
the first group including a plurality of pairs of dimer-forming honeycomb probes containing a pair of an oligonucleotide No.1 and an oligonucleotide No.2, each oligonucleotide having three regions of a 3' side region, a mid-region and a 5' side region, in which the mid-regions thereof have base sequence complementary to each other to form a dimer probe, and the 3' side regions and the 5' side regions thereof have base sequences not complementary to each other, and
the second group including a plurality of pairs of cross-linking probes containing a pair of an oligonucleotide No.3 and an oligonucleotide No.4, each oligonucleotide having two regions of a 3' side region and a 5' side region, in which the 3' side regions and the 5' side regions thereof have base sequences not complementary to each other, and the pairs of the cross-linking probes having base sequences capable of cross-linking the dimer probes formed from the dimer-forming probes; and
hybridizing the probes,
wherein the probes self-assemble to form the self-assembly substance.

5. The method according to claim 4, wherein the base sequences of the probes are complementary to each other in the following respective pairs:
the 3' side region of the oligonucleotide No.1 in the first group and the 3' side region of the oligonucleotide No.3 in the second group;
the 5' side region of the oligonucleotide No.2 in the first group and the 5' side region of the oligonucleotide No.4 in the second group;
the 3' side region of the oligonucleotide No.4 in the second group and the 3' side region of the oligonucleotide No.2 in the first group; and
the 5' side region of the oligonucleotide No.3 in the second group and the 5' side region of the oligonucleotide No.1 in the first group.

6. The method according to claim 4, wherein the base sequences of the probes are complementary to each other in the following respective pairs:
the 3' side region of the oligonucleotide No.1 in the first group and the 3' side region of the oligonucleotide No.3 in the second group;
the 5' side region of the oligonucleotide No.2 in the first group and the 5' side region of the oligonucleotide No.3 in the second group;
the 3' side region of the oligonucleotide No.2 in the first group and the 3' side region of the oligonucleotide No.4 in the second group; and
the 5' side region of the oligonucleotide No.1 in the first group and the 5' side region of the oligonucleotide No.4 in the second group.

7. The method according to any one of claims 1 to 6, wherein the capture probe is bound to a support.

8. The method according to claim 7, wherein the support is a microplate type, a slide glass type, a particle type, or an electroconductive substrate type.

9. The method according to any one of claims 1 to 8, further comprising hybridizing a labeled probe with the self-assembly substance to detect the presence of the self-assembly substance.

10. The method according to claim 9, wherein the labeled probe is a probe labeled with an enzyme of color generation type, an enzyme of luminescence generation type or a radioisotope.

11. The method according to any one of claims 1 to 8, wherein the presence of the self-assembly substance is detected by:
adding a fluorescent substance capable of binding to a nucleic acid to the self-assembly substance; and
measuring a photochemical change of the fluorescent substance.

12. The method according to any one of claims 1 to 8, wherein the presence of the self-assembly substance is detected by:
labeling in advance at least one of the honeycomb probes forming the self-assembly substance with a fluorescent substance; and
measuring a photochemical change of the fluorescent substance.

13. The method according to any one of claims 1 to 8, wherein the presence of the self-assembly substance is detected by:
labeling in advance at least one of the honeycomb probes forming the self-assembly substance with a radioisotope; and
detecting the radioisotope.

14. The method according to any one of claims 1 to 8, wherein the presence of the self-assembly substance is detected by:
labeling in advance at least one of the honeycomb probes forming the self-assembly substance with an enzyme of color generation type or an enzyme of luminescence generation type; and
measuring a photochemical change due to the enzyme.

15. The method according to any one of claims 1 to 14, wherein the honeycomb probes are comprised of at least one base selected from the group consisting of DNA, RNA, PNA, and LNA.

## Patentansprüche

1. Verfahren zum Nachweisen eines exprimierten Gens, wobei die Nachweisempfindlichkeit des exprimierten Gens auf einem DNA-Chip durch die Verwendung einer reversen Transkriptionsreaktion und einer Selbstorganisationsreaktion, die eine selbstorganisierte Substanz mittels Selbstorganisation eines Paares von in Wabenstruktur angeordneten Sonden bildet, verbessert wird, wobei das Verfahren die Schritte umfasst:
Durchführen einer reversen Transkriptionsreaktion von mRNA unter Verwendung einer ersten, am 3'-Ende poly(dT) und eine Region, die mit einer der in Wabenstruktur angeordneten Sonden hybridisieren kann, enthaltenden Sonde als Primer, um eine zweite Sonde mit einer cDNA-Region zu bilden;
Trennen der mRNA von der zweiten Sonde;
Hybridisieren der zweiten Sonde mit einer Capture-Sonde, welche eine Region aufweist, die zu einer cDNA-Region einer Ziel-mRNA komplementär ist;
Bilden einer selbstorganisierten Substanz durch eine Selbstorganisationsreaktion unter Verwendung der zweiten Sonde und der in Wabenstruktur angeordneten Sonden, wobei sich die in Wabenstruktur angeordneten Sonden selbst organisieren, um die selbstorganisierte Substanz zu bilden, und
Nachweisen der Anwesenheit der selbstorganisierten Substanz.

2. Verfahren gemäß Anspruch 1, wobei die Selbstorganisationsreaktion unter Verwendung einer Vielzahl von Paaren in Wabenstruktur angeordneter Sonden durchgeführt wird, wobei die Anzahl der zueinander komplementären Basensequenzregionen n (n ≥ 3) beträgt, derart, dass sich die in Wabenstruktur angeordneten Sonden durch abwechselndes Hybridisieren der in Wabenstruktur angeordneten Sonden aneinander selbst organisieren, um eine doppelsträngige selbstorganisierte Substanz zu bilden.

3. Verfahren zum Nachweisen eines exprimierten Gens, wobei die Nachweisempfindlichkeit des exprimierten Gens auf einem DNA-Chip durch die Verwendung einer reversen Transkriptionsreaktion und einer Selbstorganisationsreaktion, die eine selbstorganisierte Substanz mittels Selbstorganisation eines Paares von in Wabenstruktur angeordneten Sonden bildet, verbessert wird, wobei die Selbstorganisationsreaktion unter Verwendung einer Vielzahl von Paaren aus einer ersten in Wabenstruktur angeordneten Sonde ("honeycomb probe", HCP) und einer zweiten HCP durchgeführt wird, wobei die Anzahl der zueinander komplementären Basensequenzregionen n (n ≥ 3) beträgt, derart, dass sich die in Wabenstruktur angeordneten Sonden durch abwechselndes Hybridisieren der in Wabenstruktur angeordneten Sonden aneinander selbst organisieren, um eine doppelsträngige selbstorganisierte Substanz zu bilden, wobei das Verfahren die Schritte umfasst:
Binden einer ersten, am 3'-Ende poly(dT) und mindestens einen Teil der Basensequenzregionen der ersten HCP enthaltenden Sonde an mRNA;
Durchführen einer reversen Transkriptionsreaktion mit einer reversen Transkriptase, um eine zweite Sonde zu bilden, die eine cDNA-Region und mindestens einen Teil der Basensequenzregionen der ersten HCP enthält;
Entfernen der mRNA; anschließend
Hybridisieren der zweiten Sonde mit einer Capture-Sonde, welche eine Region aufweist, die zu einer cDNA-Region einer Ziel-mRNA komplementär ist;
Zugeben sowohl der ersten HCP als auch der zweiten HCP oder Zugeben der zweiten HCP, um durch die Selbstorganisationsreaktion der in Wabenstruktur angeordneten Sonden eine selbstorganisierte Substanz zu bilden, so dass Signalverstärkung erreicht werden kann, und
Nachweisen der Anwesenheit der selbstorganisierten Substanz.

4. Verfahren gemäß Anspruch 1, wobei die Selbstorganisationsreaktion die Schritte umfasst:
Bereitstellen einer ersten Gruppe und einer zweiten Gruppe Sonden,
wobei die erste Gruppe eine Vielzahl von Paaren dimerbildender, in Wabenstruktur angeordneter Sonden einschließt, die ein Paar aus einem Oligonucleotid Nr. 1 und einem Oligonucleotid Nr. 2 enthalten, wobei jedes Oligonucleotid drei Regionen aufweist, eine 3'-Seitenregion, eine mittlere Region und eine 5'-Seitenregion, wobei die mittleren Regionen desselben zueinander komplementäre Basensequenz aufweisen, um eine Dimersonde zu bilden, und die 3'-Seitenregionen und die 5'-Seitenregionen desselben zueinander nicht komplementäre Basensequenzen aufweisen, und
wobei die zweite Gruppe eine Vielzahl von Paaren quervernetzender Sonden einschließt, die ein Paar aus einem Oligonucleotid Nr. 3 und einem Oligonucleotid Nr. 4 enthalten, wobei jedes Oligonucleotid zwei Regionen aufweist, eine 3'-Seitenregion und eine 5'-Seitenregion, wobei die 3'-Seitenregionen und die 5'-Seitenregionen desselben zueinander nicht komplementäre Basensequenzen aufweisen, und die Paare aus quervernetzenden Sonden Basensequenzen aufweisen, die zum Quervernetzen der aus den dimerbildenden Sonden gebildeten Dimersonden fähig sind, und
Hybridisieren der Sonden,
wobei sich die Sonden selbst organisieren, um die selbstorganisierte Substanz zu bilden.

5. Verfahren gemäß Anspruch 4, wobei die Basensequenzen der Sonden in den folgenden jeweiligen Paaren zueinander komplementär sind:
die 3'-Seitenregion des Oligonucleotids Nr. 1 in der ersten Gruppe und die 3'-Seitenregion des Oligonucleotids Nr. 3 in der zweiten Gruppe;
die 5'-Seitenregion des Oligonucleotids Nr. 2 in der ersten Gruppe und die 5'-Seitenregion des Oligonucleotids Nr. 4 in der zweiten Gruppe;
die 3'-Seitenregion des Oligonucleotids Nr. 4 in der zweiten Gruppe und die 3'-Seitenregion des Oligonucleotids Nr. 2 in der ersten Gruppe, und
die 5'-Seitenregion des Oligonucleotids Nr. 3 in der zweiten Gruppe und die 5'-Seitenregion des Oligonucleotids Nr. 1 in der ersten Gruppe.

6. Verfahren gemäß Anspruch 4, wobei die Basensequenzen der Sonden in den folgenden jeweiligen Paaren zueinander komplementär sind:
die 3'-Seitenregion des Oligonucleotids Nr. 1 in der ersten Gruppe und die 3'-Seitenregion des Oligonucleotids Nr. 3 in der zweiten Gruppe;
die 5'-Seitenregion des Oligonucleotids Nr. 2 in der ersten Gruppe und die 5'-Seitenregion des Oligonucleotids Nr. 3 in der zweiten Gruppe;
die 3'-Seitenregion des Oligonucleotids Nr. 2 in der ersten Gruppe und die 3'-Seitenregion des Oligonucleotids Nr. 4 in der zweiten Gruppe, und
die 5'-Seitenregion des Oligonucleotids Nr. 1 in der ersten Gruppe und die 5'-Seitenregion des Oligonucleotids Nr. 4 in der zweiten Gruppe.

7. Verfahren gemäß einem der Ansprüche 1 bis 6, wobei die Capture-Sonde an eine Unterlage gebunden ist.

8. Verfahren gemäß Anspruch 7, wobei es sich bei der Unterlage um eine Art von Mikroplatte, eine Art von Objektträgerglas, eine Art von Teilchen oder eine Art von leitfähigem Substrat handelt.

9. Verfahren gemäß einem der Ansprüche 1 bis 8, ferner umfassend das Hybridisieren einer markierten Sonde mit der selbstorganisierten Substanz, um die Anwesenheit der selbstorganisierten Substanz nachzuweisen.

10. Verfahren gemäß Anspruch 9, wobei es sich bei der markierten Sonde um eine Sonde handelt, die mit einem Enzym der Farberzeugungsart, einem Enzym der Lumineszenzerzeugungsart oder einem radioaktiven Isotop markiert ist.

11. Verfahren gemäß einem der Ansprüche 1 bis 8, wobei die Anwesenheit der selbstorganisierten Substanz nachgewiesen wird durch:
Zugeben einer fluoreszierenden Substanz, die zum Binden an eine Nucleinsäure fähig ist, zu der selbstorganisierten Substanz und
Messen einer photochemischen Veränderung der fluoreszierenden Substanz.

12. Verfahren gemäß einem der Ansprüche 1 bis 8, wobei die Anwesenheit der selbstorganisierten Substanz nachgewiesen wird durch:
Markieren mindestens einer der in Wabenstruktur angeordneten Sonden, die die selbstorganisierte Substanz bilden, mit einer fluoreszierenden Substanz im Voraus und
Messen einer photochemischen Veränderung der fluoreszierenden Substanz.

13. Verfahren gemäß einem der Ansprüche 1 bis 8, wobei die Anwesenheit der selbstorganisierten Substanz nachgewiesen wird durch:
Markieren mindestens einer der in Wabenstruktur angeordneten Sonden, die die selbstorganisierte Substanz bilden, mit einem radioaktiven Isotop im Voraus und Nachweisen des radioaktiven Isotops.

14. Verfahren gemäß einem der Ansprüche 1 bis 8, wobei die Anwesenheit der selbstorganisierten Substanz nachgewiesen wird durch:
Markieren mindestens einer der in Wabenstruktur angeordneten Sonden, die die selbstorganisierte Substanz bilden, mit einem Enzym der Farberzeugungsart oder einem Enzym der Lumineszenzerzeugungsart im Voraus und
Messen einer photochemischen Veränderung auf Grund des Enzyms.

15. Verfahren gemäß einem der Ansprüche 1 bis 14, wobei die in Wabenstruktur angeordneten Sonden aus mindestens einer Base, ausgewählt aus der Gruppe, bestehend aus DNA, RNA, PNA und LNA, bestehen.

## Revendications

1. Procédé de détection d'un gène exprimé, dans lequel la sensibilité de détection du gène exprimé sur une puce à ADN est améliorée grâce à une réaction de transcription inverse et une réaction d'auto-assemblage formant une substance d'auto-assemblage par auto-assemblage d'une paire de sondes en nid d'abeille, le procédé comprenant les étapes consistant à :
effectuer une réaction de transcription inverse d'ARNm en utilisant une première sonde contenant un poly(dT) à l'extrémité 3' et une région pouvant s'hybrider à l'une des sondes en nid d'abeille en tant qu'amorce pour former une seconde sonde ayant une région d'ADNc ;
séparer l'ARNm de la seconde sonde ;
hybrider la seconde sonde à une sonde de capture ayant une région complémentaire d'une région d'ADNc d'un ARNm cible ;
former une substance d'auto-assemblage par une réaction d'auto-assemblage au moyen de la seconde sonde et des sondes en nid d'abeille, où les sondes en nid d'abeille s'auto-assemblent pour former la substance d'auto-assemblage ; et
détecter la présence de la substance d'auto-assemblage.

2. Procédé selon la revendication 1, dans lequel la réaction d'auto-assemblage est effectuée au moyen d'une pluralité de paires de sondes en nid d'abeille, où le nombre de régions de séquence de bases complémentaires les unes des autres est n (n≥3), de sorte que par hybridation des sondes en nid d'abeille les unes aux autres en alternance, les sondes en nid d'abeille s'auto-assemblent pour former une substance d'auto-assemblage double brin.

3. Procédé de détection d'un gène exprimé, dans lequel la sensibilité de détection du gène exprimé sur une puce à ADN est améliorée grâce à une réaction de transcription inverse et une réaction d'auto-assemblage formant une substance d'auto-assemblage par auto-assemblage d'une paire de sondes en nid d'abeille, où la réaction d'auto-assemblage est effectuée au moyen d'une pluralité de paires d'une première sonde en nid d'abeille (SNA) et d'une seconde SNA, où le nombre de régions de séquence de bases complémentaires les unes des autres est n (n≥3), de sorte que par hybridation des sondes en nid d'abeille les unes aux autres en alternance, les sondes en nid d'abeille s'auto-assemblent pour former une substance d'auto-assemblage double brin, le procédé comprenant les étapes consistant à :
lier une première sonde contenant un poly(dT) à l'extrémité 3' et au moins une partie des régions de séquence de bases de la première SNA à un ARNm ;
effectuer une réaction de transcription inverse par une transcriptase inverse pour former une seconde sonde contenant une région d'ADNc et au moins une partie des régions de séquence de bases de la première SNA ;
éliminer l'ARNm ; puis
hybrider la seconde sonde à une sonde de capture ayant une région complémentaire d'une région d'ADNc d'un ARNm cible ;
ajouter à la fois la première SNA et la seconde SNA ou ajouter la seconde SNA pour former une substance d'auto-assemblage par la réaction d'auto-assemblage des sondes en nid d'abeille de façon à pouvoir obtenir une amplification du signal, et
détecter la présence de la substance d'auto-assemblage.

4. Procédé selon la revendication 1, dans lequel la réaction d'auto-assemblage comprend les étapes consistant à :
disposer d'un premier groupe et d'un second groupe de sondes,
le premier groupe comprenant une pluralité de paires de sondes en nid d'abeille formant un dimère, contenant une paire d'un oligonucléotide n° 1 et d'un oligonucléotide n° 2, chaque oligonucléotide ayant trois régions parmi une région du côté 3', une région centrale et une région du côté 5', où les régions centrales desdits oligonucléotides ont des séquences de bases complémentaires entre elles pour former une sonde dimère, et les régions du côté 3' et les régions du côté 5' desdits oligonucléotides ont des séquences de bases qui ne sont pas complémentaires entre elles, et
le second groupe comprenant une pluralité de paires de sondes de réticulation contenant une paire d'un oligonucléotide n° 3 et d'un oligonucléotide n° 4, chaque oligonucléotide ayant deux régions parmi une région du côté 3' et une région du côté 5', où les régions du côté 3' et les régions du côté 5' desdits oligonucléotides ont des séquences de bases qui ne sont pas complémentaires entre elles, et les paires des sondes de réticulation ont des séquences de bases capables de réticuler les sondes dimères formées à partir des sondes formant un dimère ; et
hybrider les sondes,
où les sondes s'auto-assemblent pour former la substance d'auto-assemblage.

5. Procédé selon la revendication 4, dans lequel les séquences de bases des sondes sont complémentaires entre elles dans les paires respectives suivantes :
la région du côté 3' de l'oligonucléotide n° 1 dans le premier groupe et la région du côté 3' de l'oligonucléotide n° 3 dans le second groupe ;
la région du côté 5' de l'oligonucléotide n° 2 dans le premier groupe et la région du côté 5' de l'oligonucléotide n° 4 dans le second groupe ;
la région du côté 3' de l'oligonucléotide n° 4 dans le second groupe et la région du côté 3' de l'oligonucléotide n° 2 dans le premier groupe ; et
la région du côté 5' de l'oligonucléotide n° 3 dans le second groupe et la région du côté 5' de l'oligonucléotide n° 1 dans le premier groupe.

6. Procédé selon la revendication 4, dans lequel les séquences de bases des sondes sont complémentaires entre elles dans les paires respectives suivantes :
la région du côté 3' de l'oligonucléotide n° 1 dans le premier groupe et la région du côté 3' de l'oligonucléotide n° 3 dans le second groupe ;
la région du côté 5' de l'oligonucléotide n° 2 dans le premier groupe et la région du côté 5' de l'oligonucléotide n° 3 dans le second groupe ;
la région du côté 3' de l'oligonucléotide n° 2 dans le premier groupe et la région du côté 3' de l'oligonucléotide n° 4 dans le second groupe ; et
la région du côté 5' de l'oligonucléotide n° 1 dans le premier groupe et la région du côté 5' de l'oligonucléotide n° 4 dans le second groupe.

7. Procédé selon l'une quelconque des revendications 1 à 6, dans lequel la sonde de capture est liée à un support.

8. Procédé selon la revendication 7, dans lequel le support est un type de microplaque, un type de lame de verre, un type de particule, ou un type de substrat électroconducteur.

9. Procédé selon l'une quelconque des revendications 1 à 8, comprenant en outre l'hybridation d'une sonde marquée à la substance d'auto-assemblage pour détecter la présence de la substance d'auto-assemblage.

10. Procédé selon la revendication 9, dans lequel la sonde marquée est une sonde marquée avec une enzyme du type à génération de couleur, une enzyme du type à génération de luminescence ou un radioisotope.

11. Procédé selon l'une quelconque des revendications 1 à 8, dans lequel la présence de la substance d'auto-assemblage est détectée par :
ajout d'une substance fluorescente capable de se lier à un acide nucléique à la substance d'auto-assemblage : et
mesure d'un changement photochimique de la substance fluorescente.

12. Procédé selon l'une quelconque des revendications 1 à 8, dans lequel la présence de la substance d'auto-assemblage est détectée par :
marquage à l'avance d'au moins une des sondes en nid d'abeille formant la substance d'auto-assemblage, avec une substance fluorescente ; et
mesure d'un changement photochimique de la substance fluorescente.

13. Procédé selon l'une quelconque des revendications 1 à 8, dans lequel la présence de la substance d'auto-assemblage est détectée par :
marquage à l'avance d'au moins une des sondes en nid d'abeille formant la substance d'auto-assemblage, avec un radioisotope ; et
détection du radioisotope.

14. Procédé selon l'une quelconque des revendications 1 à 8, dans lequel la présence de la substance d'auto-assemblage est détectée par :
marquage à l'avance d'au moins une des sondes en nid d'abeille formant la substance d'auto-assemblage, avec une enzyme du type à génération de couleur ou une enzyme du type à génération de luminescence ; et
mesure d'un changement photochimique dû à l'enzyme.

15. Procédé selon l'une quelconque des revendications 1 à 14, dans lequel les sondes en nid d'abeille sont constituées d'au moins une base choisie dans le groupe constitué d'un ADN, d'un ARN, d'un acide nucléique peptidique (PNA) et d'un acide nucléique verrouillé (LNA).
